# EUROPEAN PATENT APPLICATION

(11) **EP 4 388 993 A1**
(43) Date of publication of application: **26.06.2024**
(21) Application number: 22215620.0
(22) Date of filing: 21.12.2022
(51) Int. Cl.: A61B 5/024, A61B 5/263, A61B 5/256, A61B 5/00

(54) **AMBIENT-LIGHT SEAL WITH INTEGRATED DRY ELECTRODES FOR ELECTRICAL VITAL SIGNS MEASUREMENTS**

(71) Applicant: Koninklijke Philips N.V., 5656 AG Eindhoven (NL)
(72) Inventor: MEFTAH, Mohamed, Eindhoven (NL); GELISSEN, Jozef Hubertus, Eindhoven (NL)
(74) Representative: Philips Intellectual Property & Standards

(57) **Abstract**

A wearable device (10) for individual health assessment measurement of vital signs comprises a housing (16), an optical sensor/emitter assembly (18), a controller (20), and a light seal (12). The optical sensor/emitter assembly emits optical signals from a bottom surface (22) of the housing through skin of a subject and receives reflected optical signals. The controller (20) controls a physiological measurement modality of the wearable device in the measurement of the vital signs and is coupled to the optical sensor/emitter assembly (18) for controlling the optical sensor/emitter assembly. The light seal (12) is configured to be removably coupled with the skin of the subject and to prevent ambient light interference with the optical sensor/emitter assembly when coupled. The light seal (12) comprises at least one integrated dry electrode (14) for transmitting and/or receiving electrical signals to/from the skin of the subject. Each integrated dry electrode (14) is electrically coupled to the controller (20) for obtaining electrical measurements according to the physiological measurement modality.

## Description

### BACKGROUND

The present embodiments relate generally to wearable devices and more particularly, to wearable devices having an ambient-light seal with integrated dry electrodes for electrical vital signs measurements.

Wrist-worn health monitoring devices (e.g., Health watches) enable users to track their health 24/7 and have become more and more popular. Nowadays, these wearables make use of multiple physiological measurement modalities (e.g., electrocardiography (ECG), photoplethysmography (PPG), Bioelectrical impedance analysis (BIA)) to obtain physiological parameters such as heart rate (HR) or pulse rate (PR), respiration rate (RR), SpO₂, stress, etc. Even though the number of sensors integrated in the watch is growing, it is important that the watch stays small and convenient to wear.

In addition, an ongoing trend in Health watches is the extension of the number of vital signs measurements to enable better and holistic health assessment. However, the small form factor of the watch limits the number of new transducers that can be integrated (i.e., the watch should not become too bulky/uncomfortable). Another constraint is that a preferred wrist location for a new transducer (e.g., for optimal signal-to-noise ratio (SNR)) may already be "occupied" by other components.
Accordingly, an improved method and apparatus for overcoming the problems in the art is desired.

### SUMMARY

An ongoing trend in Health watches is the extension of the number of vital signs measurements to enable better and holistic health assessment. However, the small form factor of the watch limits the number of transducers that can be integrated. In addition, Health watches make use of a so-called Light Seal to shield the optical sensor from ambient light interference in order to derive a reliable PPG-based signal such as heart rate and SpOz. In addition, the Light Seal ensures a comfortable and stable coupling of the watch to the wrist. The embodiments of the present disclosure include a novel Light Seal with integrated dry electrodes to advantageously enable electrical vital signs measurements such as Bioelectrical Impedance Analysis (BIA), Electrocardiography (ECG) and Galvanic Skin Response (GSR). Key elements of the Light Seal according to the embodiments of the present disclosure are the dry electrode material and the configuration of at least one electrode to make reliable and comfortable measurements possible without impacting a current ambient-light shielding functionality and form factor of the Light Seal.

According to one embodiment, a wearable device for individual health assessment measurement of vital signs comprises a housing having a bottom surface and an optical sensor/emitter assembly configured for emitting optical signals from the bottom surface of the housing through the skin of a subject and receiving reflected optical signals. A controller controls at least one physiological measurement modality of the wearable device in the measurement of the vital signs, wherein the controller is coupled to the optical sensor/emitter assembly for controlling the optical sensor/emitter assembly in transmitting and receiving optical signals. A light seal is configured to be removably coupled with the skin of the subject and to prevent ambient light interference with the optical sensor/emitter assembly when coupled. The light seal comprises at least one integrated dry electrode for at least one of (i) transmitting electrical signals to, (ii) receiving electrical signals from, and (iii) both transmitting and receiving electrical signals to/from the skin of the subject, the at least one integrated dry electrode being electrically coupled to the controller for obtaining electrical measurements according to the at least one physiological measurement modality during operation of the wearable device.

In another embodiment, the light seal comprises a physical configuration positioned about and extending from an outer perimeter of the bottom surface of the housing, wherein the light seal further includes a sidewall height dimension for spacing the optical sensor/emitter assembly with respect to the skin of the subject. According to another embodiment, the at least one integrated dry electrode comprises multiple integrated dry electrodes, each separated from one another via electrically insulative material or an air gap. In one embodiment, one or more integrated dry electrode comprises a semi-circular shape when viewed from the bottom surface of the housing. A semi-circular shape is preferred, however other shapes may be used. For example, any suitable electrode geometry/shape that combines the function of ambient light shield and bio-electrode(s) may be used. In yet another embodiment, the light seal may comprise a rubber ring integrated with at least one dry electrode that comprises a flexible, conductive polymer or elastomer.

According to another embodiment, the at least one physiological measurement modalities include one selected from the group consisting of electrocardiography (ECG), photoplethysmography (PPG), bioelectrical impedance analysis (BIA), and any combination thereof, to obtain physiological parameters that include one selected from the group consisting of heart rate (HR), pulse rate (PR), respiration rate (RR), SpO₂, stress, and any combination thereof. In another embodiment, the optical sensor/emitter assembly comprises a photoplethysmography PPG assembly for deriving a PPG-based signal that includes heart rate HR and oxygen saturation SpOz.

In one embodiment, the wearable device further comprises a wearable band or patch coupled to the housing for securing the housing to the skin of the subject while the wearable device is being worn by the subject. The wearable band may comprise one selected from the group consisting of a head band, wrist band, an ankle band, a chest band, and a waist band.

In another embodiment, the wearable device further comprises at least one additional dry electrode coupled to a side surface of the housing, other than the bottom surface, wherein the at least one additional dry electrode is electrically coupled to the controller for obtaining electrical measurements according to the at least one physiological measurement modality during operation of the wearable device. The at least one additional dry electrode may comprise two additional dry electrodes, wherein the two additional dry electrodes are disposed on a same side surface of the housing.

In one embodiment, a wearable device for individual health assessment measurement of vital signs comprises a housing, an optical sensor/emitter assembly, a controller, and a light seal. The optical sensor/emitter assembly is configured for emitting optical signals from a bottom surface of the housing through skin of a subject and receiving reflected optical signals. The controller may control at least one physiological measurement modality of the wearable device in the measurement of the vital signs, wherein the controller is coupled to the optical sensor/emitter assembly for controlling the optical sensor/emitter assembly in transmitting and receiving of optical signals. The light seal is configured to be removably coupled with the skin of the subject and to prevent ambient light interference with the optical sensor/emitter assembly when coupled. The light seal comprises at least one integrated dry electrode for at least one of (i) transmitting electrical signals to, (ii) receiving electrical signals from, and (iii) both transmitting and receiving electrical signals to/from the skin of the subject. The at least one integrated dry electrode may be electrically coupled to the controller for obtaining electrical measurements according to the at least one physiological measurement modality during operation of the wearable device.

In addition, the light seal may comprise a physical configuration positioned about and extending from an outer perimeter of the bottom surface of the housing, wherein the light seal further includes a sidewall height dimension for spacing the optical sensor/emitter assembly with respect to the skin of the subject. The at least one physiological measurement modalities include one selected from the group consisting of electrocardiography (ECG), photoplethysmography (PPG), bioelectrical impedance analysis (BIA), and any combination thereof, to obtain physiological parameters that include one selected from the group consisting of heart rate (HR), pulse rate (PR), respiration rate (RR), SpO2, stress, and any combination thereof.

In another embodiment, the optical sensor/emitter assembly comprises a photoplethysmography PPG assembly for deriving a PPG-based signal that includes heart rate HR and oxygen saturation SpO₂. The at least one integrated dry electrode may comprise multiple integrated dry electrodes, each separated from one another via electrically insulative material. In another embodiment, one or more integrated dry electrode may comprise a semi-circular shape when viewed from the bottom surface of the housing. According to another embodiment, the light seal may comprise a rubber ring integrated with at least one dry electrode that comprises a flexible, conductive polymer or elastomer.

According to yet another embodiment, the wearable device further comprises a wearable band or patch coupled to the housing for securing the housing to the skin of the subject while the wearable device is being worn by the subject. The wearable band may comprise one selected from the group consisting of a head band, wrist band, an ankle band, a chest band, and a waist band.

In one embodiment, the wearable device may further comprise at least one additional dry electrode coupled to a side surface of the housing, other than the bottom surface, wherein the at least one additional dry electrode is electrically coupled to the controller for obtaining electrical measurements according to the at least one physiological measurement modality during operation of the wearable device. In another embodiment, the at least one additional dry electrode comprises two additional dry electrodes, wherein the two additional dry electrodes are disposed on a same side surface of the housing.

The embodiments of the present disclosure advantageously integrate dry electrodes into a Health watch to enable electrical vital signs measurements, such as Bioelectrical Impedance Analysis (BIA), Electrocardiography (ECG) and Galvanic Skin Response (GSR), without affecting the form factor.

Another advantage resides in use of a so-called Light Seal to shield the optical sensor from ambient light interference in order to derive a reliable PPG-based measurement such as heart rate and SpO₂. In addition, a Light Seal in the form of a rubber ring combines the above-mentioned functionality and dry electrodes, according to the embodiments of the present disclosure, which advantageously allows for and ensures a comfortable and stable coupling of the wearable device to the wrist and also enables electrical vital signs measurements, such as Bioelectrical Impedance Analysis (BIA), Electrocardiography (ECG) and Galvanic Skin Response (GSR), via the light seal.

Still further advantages and benefits will become apparent to those of ordinary skill in the art upon reading and understanding the following detailed description.

### BRIEF DESCRIPTION OF THE DRAWINGS

The embodiments of the present disclosure may take form in various components and arrangements of components, and in various steps and arrangements of steps. Accordingly, the drawings are for purposes of illustrating the various embodiments and are not to be construed as limiting the embodiments. In the drawing Figs, like reference numerals refer to like elements. In addition, it is to be noted that the Figs may not be drawn to scale.
Fig. 1 is a block diagram view of a wearable device for individual health assessment measurement of vital signs including a light seal that comprises at least one integrated dry electrode according to an embodiment of the present disclosure;
Fig. 2 is a schematic perspective view of a wearable device for individual health assessment measurement of vital signs including a light seal that comprises at least one integrated dry electrode according to an embodiment of the present disclosure;
Fig. 3 is another schematic perspective view of a wearable device for individual health assessment measurement of vital signs including a light seal that comprises at least one integrated dry electrode according to an embodiment of the present disclosure illustrating mounting placement of the light seal to a bottom surface of the wearable device housing;
Fig. 4 is a bottom view of the wearable device including a light seal that comprises at least one integrated dry electrode according to an embodiment of the present disclosure;
Fig. 5 is a block diagram view of a wearable device for individual health assessment measurement of vital signs including a light seal that comprises two integrated dry electrodes according to an embodiment of the present disclosure;
Fig. 6 is a bottom view of the wearable device including a light seal that comprises at least one integrated dry electrode according to an embodiment of the present disclosure; and
Fig. 7 is a schematic perspective view of a wearable device for individual health assessment measurement of vital signs including a light seal that comprises at least one integrated dry electrode according to an embodiment of the present disclosure.

### DETAILED DESCRIPTION

The embodiments of the present disclosure and the various features and advantageous details thereof are explained more fully with reference to the non-limiting examples that are described and/or illustrated in the drawings and detailed in the following description. It should be noted that the features illustrated in the drawings are not necessarily drawn to scale, and features of one embodiment may be employed with other embodiments as the skilled artisan would recognize, even if not explicitly stated herein. Descriptions of well-known components and processing techniques may be omitted so as to not unnecessarily obscure the embodiments of the present disclosure. The examples used herein are intended merely to facilitate an understanding of ways in which the embodiments of the present may be practiced and to further enable those of skill in the art to practice the same. Accordingly, the examples herein should not be construed as limiting the scope of the embodiments of the present disclosure, which is defined solely by the appended claims and applicable law.

It is understood that the embodiments of the present disclosure are not limited to the particular methodology, protocols, devices, apparatus, materials, applications, etc., described herein, as these may vary. It is also to be understood that the terminology used herein is used for the purpose of describing particular embodiments only, and is not intended to be limiting in scope of the embodiments as claimed. It must be noted that as used herein and in the appended claims, the singular forms "a," "an," and "the" include plural reference unless the context clearly dictates otherwise.

Unless defined otherwise, all technical and scientific terms used herein have the same meanings as commonly understood by one of ordinary skill in the art to which the embodiments of the present disclosure belong. Preferred methods, devices, and materials are described, although any methods and materials similar or equivalent to those described herein can be used in the practice or testing of the embodiments.

According to one embodiment, key elements to the wearable device are dry electrode material and a configuration of at least one electrode to make reliable and comfortable measurements possible without impacting a current ambient-light shielding functionality and form factor of the Light Seal. In addition, the wearable device according to the embodiments of the present disclosure is based on a new hardware component combined with existing and/or slightly modified algorithms, e.g., to extract an ECG signal.

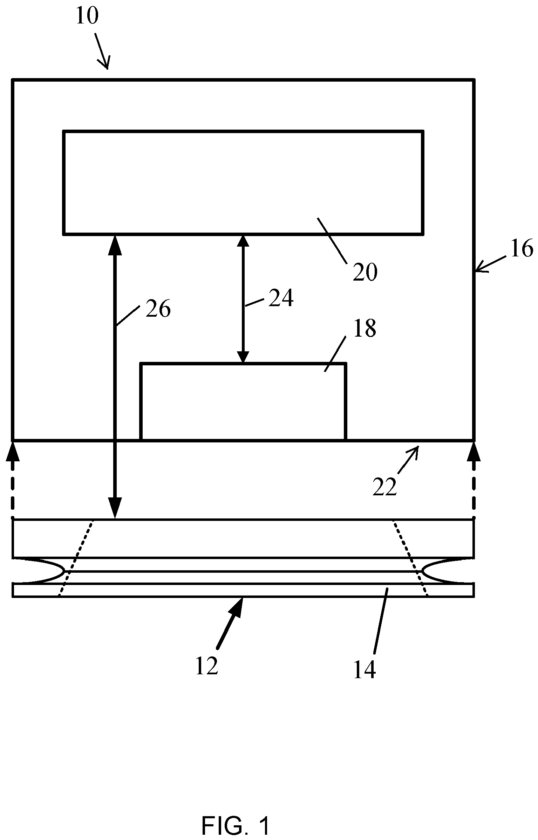

With reference now to Fig. 1, a block diagram view of a wearable device 10 for individual health assessment measurement of vital signs is shown including a light seal 12 that comprises at least one integrated dry electrode 14 according to an embodiment of the present disclosure. The wearable device 10 for individual health assessment measurement of vital signs comprises a housing 16, an optical sensor/emitter assembly 18, a controller 20, and the light seal 12. The optical sensor/emitter assembly 18 is configured for emitting optical signals from a bottom surface 22 of the housing through skin of a subject (not shown) and receiving reflected optical signals. The controller 20 may control at least one physiological measurement modality of the wearable device 10 in the measurement of the vital signs. The controller 20 is coupled, via one or more signal line(s) 24, to the optical sensor/emitter assembly 18 for controlling the optical sensor/emitter assembly in transmitting and receiving of optical signals.

In one embodiment, controller 20 comprises one or more of a microprocessor, microcontroller, field programmable gate array (FPGA), integrated circuit, discrete analog or digital circuit components, hardware, software, firmware, or any combination thereof, for performing various functions as discussed herein, further according to the requirements of a given wearable device for health assessment measurement of vital signs (or health watch) implementation and/or application. Controller 20 may further comprise one or more of the various modules. In one embodiment, the modules may comprise one or more of an integrated circuit, discrete analog or digital circuit components, hardware, software, firmware, or any combination thereof, for performing various functions as discussed herein, further according to the requirements of a given wearable device for health assessment implementation and/or application. In addition, one or more of the modules can further comprise various combinations of one or more of the various modules. Additional details regarding the controller 20 and modules will be provided herein below with reference to the Figs.

The light seal 12 is configured to be removably coupled with the skin of the subject (not shown) and to prevent ambient light interference with the optical sensor/emitter assembly 18 when coupled. The light seal 12 comprises at least one integrated dry electrode 14 for at least one of (i) transmitting electrical signals to, (ii) receiving electrical signals from, and (iii) both transmitting and receiving electrical signals to/from the skin of the subject. The at least one integrated dry electrode 14 may be electrically coupled, via one or more signal line(s) 26, to the controller 20 for obtaining electrical measurements according to the at least one physiological measurement modality during operation of the wearable device 10. The light seal 12 is physically coupled to the bottom surface 22 of the housing 16, via a suitable mounting method according to the requirements of a given wearable device health watch implementation. Suitable mounting methods may include gluing, bonding, press-fitting, as well as, other methods, (e.g., screws, magnets, etc.). In addition, in one embodiment, the physical mounting of light seal 12 to the housing 16 includes electrical coupling, as appropriate, between the controller 20 and the at least one dry electrode 14.

In one embodiment, the light seal 12 with an integrated dry electrode 14 includes an electrode body configured to be removably coupled with the skin of a subject and to receive electrical signals from and/or transmit electrical signals to skin of subject. The light seal 12 further includes an electrical coupling that facilitates coupling the dry electrode 14 to controller 20, via signal line(s) 26 (Fig. 1). The electrode body includes a conductive silicone material configured to enable uptake or diffusion of moisture from skin of the subject over which the electrode body is disposed; and a detergent configured to facilitate a flow of ions through the conductive silicone material. In one embodiment, conductive particles fill the dry silicone electrode with detergent additives for optimal sensing electrical contact with skin of the subject. Dry electrode 14 is soft and skin friendly due to the hydrophilic silicone material having moisture regulating properties. In one embodiment, the light seal 12 comprises a rubber ring integrated with at least one dry electrode 14 that comprises a flexible, conductive polymer or elastomer.

In some embodiments, the electrode body includes silicone material, electrically conductive particles, and detergent. In other embodiments, the electrically conductive particles may include graphite, carbon or silver flakes or particles. In some embodiments, the electrically conductive particles are mixed with the silicone material to form a conductive silicone material. In some embodiments, the electrode body may include a range of about 25 to 75 % weight of the silicone material, a range of about 20 to 50 % weight of the conductive particles and a range of about 5 to 25 % weight of the detergent. In some embodiments, the electrode body may include 50 % weight of the silicone material, 35 % weight of the conductive particles and 15 % weight of the detergent. In some embodiments, the silicone material, the conductive particles and the detergent are mixed using mix-extrusion, high speed mixing process, which is performed at temperature ranges of about 10 to 150°C. In some embodiments, the silicone material, the conductive particles and the detergent are mixed using a high-speed mixing or mix extrusion device.

In some embodiments, additional materials (additives) may be added to facilitate mixing of the conductive silicone material and the detergent and/or mixing the silicone material, the conductive particles and the detergent. In some embodiments, such additional materials may be optional. In some embodiments, such additional materials do not change the impedance of the dry electrode formed from the hydrophilic silicone material and the detergent additives.

In some embodiments, the conductive silicone material is configured to provide moisture regulating properties to dry electrode 14. In some embodiments, the conductive silicone material is a hydrophilic conductive silicone material or a PolyDiMethylSiloxane material. The added hydrophilic properties of the conductive silicone material cause moisture uptake from the subject's skin and from the air surrounding the subject's skin. Thus, the conductive silicone material of the electrode provides a comfortable layer against the skin. In some embodiments, the conductive silicone material may include electrically conductive liquid silicone rubber. In some embodiments, the conductive silicone material may include commercially available Elastosil^{®} LR 3162 material.

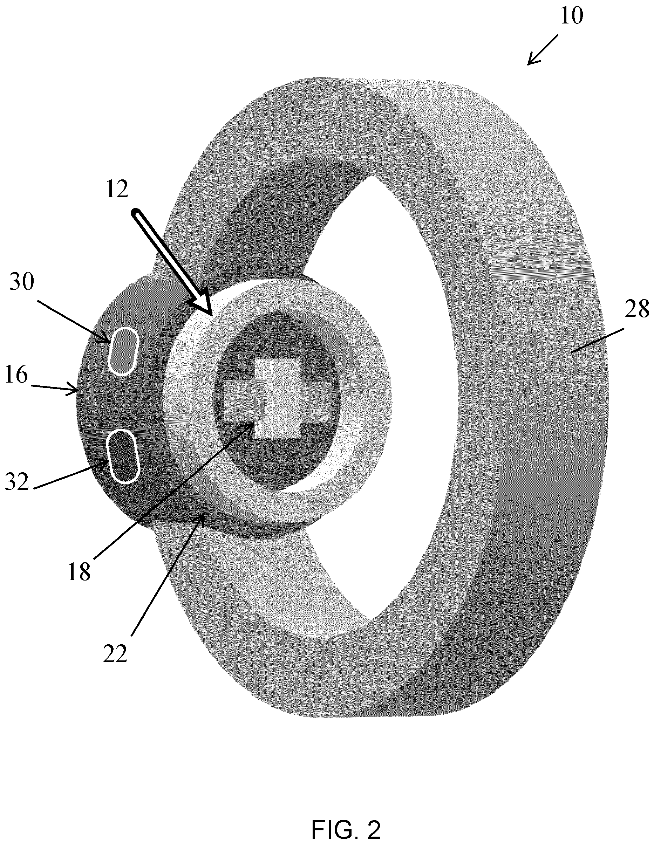

With reference now to Fig. 2, a schematic perspective view of the wearable device 10 for individual health assessment measurement of vital signs is shown. The wearable device 10 includes a light seal 12 that comprises at least one integrated dry electrode 14 according to an embodiment of the present disclosure. The perspective view also illustrates a wrist band 28 coupled to the housing 16, wherein the wrist band 28 is used to removably secure the wearable device 10 to the wrist (not shown) of a subject when being worn by the subject. Wrist band 28 could include any suitable material and latch mechanism (not shown) for adjusting how tight the wearable device is held to the subject's wrist. In another embodiment, a wearable band or patch (not shown) is coupled to the housing 16 for securing the housing 16 to the skin of the subject while the wearable device 10 is being worn by the subject. In yet another embodiment, the wearable band comprises one selected from the group consisting of a head band, wrist band, an ankle band, a chest band, and a waist band.

Wearable device 10 may further include auxiliary electrodes 30 and 32 positioned on an outer side surface of housing 16. In one embodiment, the auxiliary electrodes comprise at least one additional dry electrode coupled to a side surface of the housing, other than the bottom surface, wherein the at least one additional dry electrode is electrically coupled to the controller for obtaining electrical measurements according to the at least one physiological measurement modality during operation of the wearable device. In one embodiment, the at least one additional dry electrode comprises two additional dry electrodes, wherein the two additional dry electrodes are disposed on a same side surface of the housing 16.

In the illustration of Fig. 2, according to one embodiment, the light seal 12 has an outer perimeter surface that is at a diameter which is less than a diameter of a corresponding outer perimeter surface of the housing 16 upon which the light seal is mounted. In another embodiment, as shown in Fig. 1, the outer perimeter surface of the light seal 12 is aligned with the outer perimeter surface of the housing 16. In addition, the light seal 12 has a height dimension which is configured for spacing the sensor/emitter assembly 18 at a given height with respect to the skin surface of subject during use of wearable device 10 while worn by the subject. For example, spacing of the sensor/emitter assembly may correspond to a spacing for locating the sensor/emitter assembly at the skin surface, above the skin surface, or pressed into the skin surface, according to the requirements of a given health watch implementation. One health watch implementation may include the optical sensor/emitter being placed in contact with the skin. A non-skin contact option may also be possible.

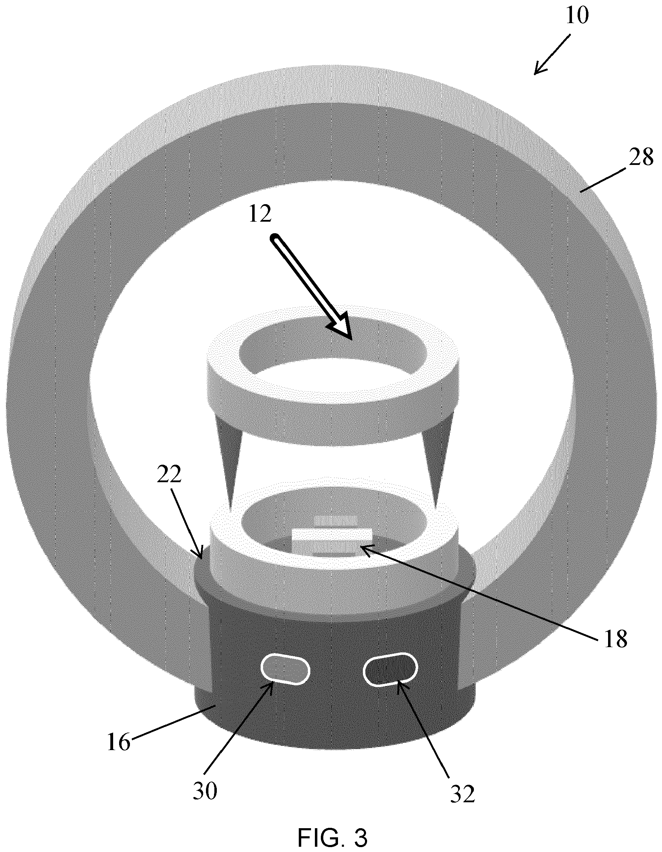

Turning now to Fig 3, another schematic perspective view is shown of a wearable device 10 for individual health assessment measurement of vital signs including a light seal 12 that comprises at least one integrated dry electrode 14 according to an embodiment of the present disclosure. Fig. 3 illustrates one possible mounting placement of the light seal 12 to a bottom surface 22 of the wearable device housing 16.

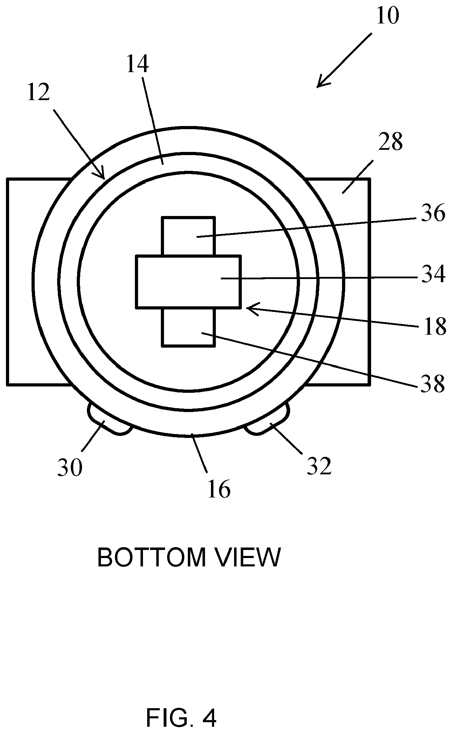

With reference now to Fig. 4, there is shown a bottom view of the wearable device 10 including a light seal 12 that comprises at least one integrated dry electrode 14 according to an embodiment of the present disclosure. The sensor/emitter assembly 18 includes a sensor 34 and emitters 36, 38 disposed on opposite sides of the sensor 34. In one embodiment, the sensor/emitter assembly 18 is configured for obtaining an optical measurement (e.g., SpO₂) that requires the light seal 12 to shield the optical sensor 34 from ambient light interference in order to derive a reliable PPG-based measurement, such as heart rate and oxygen saturation SpOz. For example, SpO₂, also known as oxygen saturation, is a measure of the amount of oxygen-carrying hemoglobin in the blood relative to the amount of hemoglobin not carrying oxygen. The body needs there to be a certain level of oxygen in the blood or it will not function as efficiently. In fact, very low levels of SpO2 can result in very serious symptoms. This condition is known as hypoxemia. There is a visible effect on the skin, known as cyanosis due to the blue (cyan) tint it takes on. Hypoxemia (low levels of oxygen in the blood) can turn into hypoxia (low levels of oxygen in the tissue). This progression and the difference between the two conditions is important to understand.

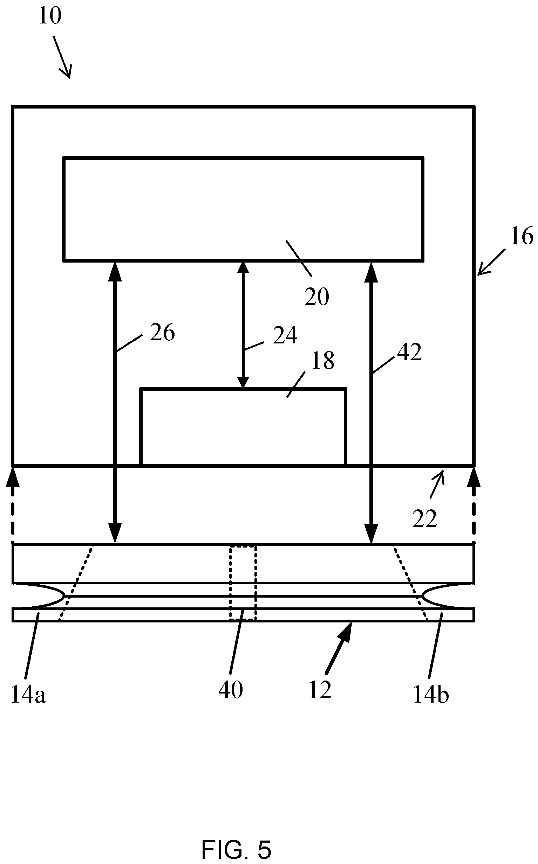

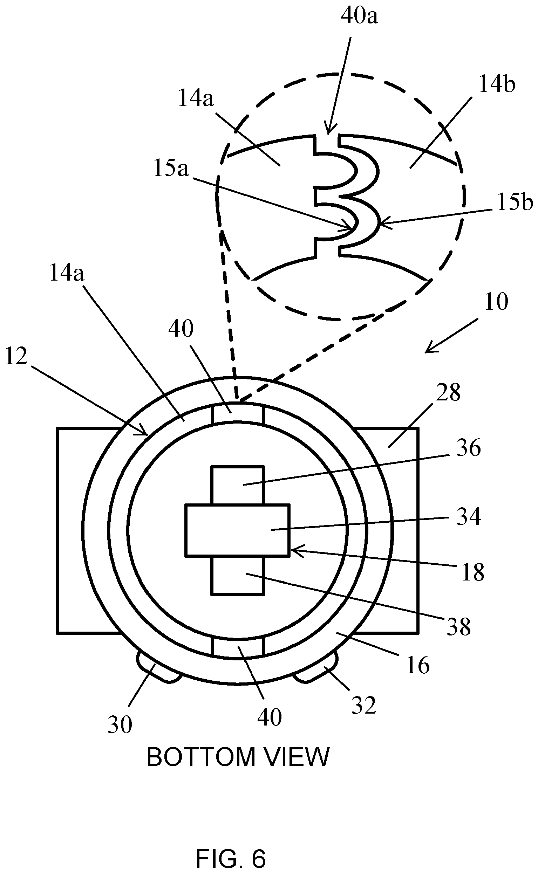

In Fig. 5, a block diagram view is shown of a wearable device 10 for individual health assessment measurement of vital signs including a light seal 12 that comprises two integrated dry electrodes 14a, 14b according to an embodiment of the present disclosure. In one embodiment, the integrated dry electrodes may each comprise a semi-circular shape when viewed from the bottom surface of the housing. A semi-circular shape is preferred, however other shapes may be used. For example, any suitable electrode geometry/shape that combines the function of ambient light shield and bio-electrode(s) may be used. In addition, the integrated dry electrodes 14a, 14b are each separated from one another via electrically insulative material or insulator 40. Examples of suitable insulative material may include silicone rubber, plastic, or glass. In another embodiment, the insulator 40 may comprise an air gap 40a as an electrical insulator (See Fig. 6, i.e., dashed circle insert), wherein the air gap 40a is disposed between sidewall surfaces (15a, 15b) of adjacent dry electrodes (14a, 14b), respectively, in a region of the air gap 40a, further wherein the air gap and a configuration of the sidewall surfaces of the adjacent dry electrodes are adapted to prevent entry or passage of ambient light through the air gap. In other words, ambient light outside the light seal is prevented, by virtue of the air gap and sidewall surface configurations of adjacent dry electrodes, from traversing the air gap from an exterior of the light seal to an interior of the light seal in a region of the sensor/emitter assembly 18. The sidewall surface configurations may be complementary of one another, e.g., as shown in Fig. 6. Alternatively, the sidewall surface configurations may be other than complementary of one another, but still prevent ambient light passage through the air gap 40a. The light seal 12 further includes an electrical coupling that facilitates coupling the dry electrodes 14a and 14b to controller 20, via signal line(s) 26 and 42, respectively.

According to one embodiment, the at least one dry electrode 14 is integrated in the light seal 12 of the wearable device (or Health watch) 10 to enable Bioelectrical Impedance Analysis (BIA). BIA is a simple and non-invasive method for estimating body composition (e.g., body fat), by measuring the electrical impedance of a human body. With reference now to Figs 5 and 6, the technique is based on a 4-points measurement wherein 2 electrodes (e.g., auxiliary electrodes 30, 32) are used for electrical current injection through the body and 2 electrodes for voltage measurement (e.g., dry electrodes 14a, 14b). To conduct the BIA, the subject needs to touch the 2 electrodes (e.g., auxiliary electrodes 30, 32) located at the side of the wearable drive 10 or health watch, while the 2 bottom electrodes (e.g., dry electrodes 14a, 14b) should be in contact with the subject's wrist while the wearable device 10 is being worn by the subject. As shown in Fig. 6, one or more integrated dry electrode 14a, 14b comprises a semi-circular shape when viewed from the bottom surface of the housing 16.

The embodiments of the present disclosure are beneficial when BIA is combined with an optical measurement (e.g., SpOz) that requires the light seal 12. Figs 5 and 6 shows an example of how the implementation could be implemented. In this case, the light seal 12 is made of two dry electrodes (14a and 14b) with an electrical insulating material or insulator 40 in between. To ensure high user comfort during long-term wear, it is preferrable to use dry electrodes 14a, 14b that are based on a soft/flexible material or materials such as conductive polymers/elastomer. The dry electrodes do not require skin preparation and can be easily cleaned. The dry electrodes can be customized according to the desired design.

It is noted that, although the embodiments have been explained for BIA, the embodiments may also be applicable to other applications and parameters of interest. Such other applications and parameters of interest may include, for example, Electrocardiography (ECG), Galvanic Skin Response (GSR), etc. The number, size and position of the dry electrodes can be defined and configured, according to the particular wearable device health watch implementation.

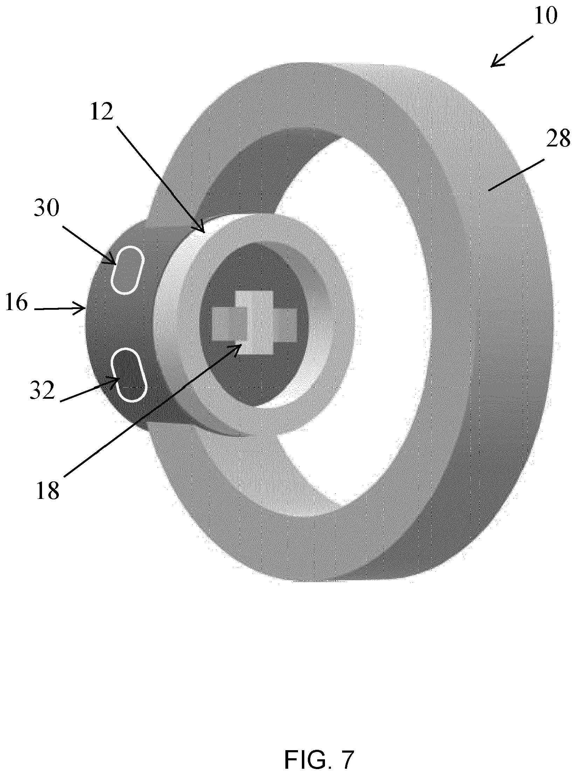

With reference now to Fig. 7, a schematic perspective view of the wearable device 10 for individual health assessment measurement of vital signs is shown. The wearable device 10 includes a light seal 12 that comprises at least one integrated dry electrode 14 according to an embodiment of the present disclosure. The perspective view also illustrates a wrist band 28 coupled to the housing 16, wherein the wrist band 28 is used to removably secure the wearable device 10 to the wrist (not shown) of a subject when being worn by the subject. Wrist band 28 could include any suitable wrist band latch mechanism (not shown) for adjusting how tight the wearable device is held to the subject's wrist. Wearable device 10 may further include auxiliary electrodes 30 and 32 positioned on an outer side surface of housing 16. In the illustration of Fig. 7, according to one embodiment, the light seal 12 has an outer perimeter surface that is at a diameter which is aligned with and equal to a diameter of a corresponding outer perimeter surface of the housing 16 upon which the light seal is mounted. Such a configuration is also as shown in Fig. 1, wherein the outer perimeter surface of the light seal 12 is aligned with the outer perimeter surface of the housing 16. In addition, the light seal 12 has a height dimension which is configured for spacing the sensor/emitter assembly 18 at a given height above and/or from the skin surface of subject during use of wearable device 10 while worn by the subject.

Although only a few exemplary embodiments have been described in detail above, those skilled in the art will readily appreciate that many modifications are possible in the exemplary embodiments without materially departing from the novel teachings and advantages of the embodiments of the present disclosure. For example, the embodiments of the present disclosure may be advantageously used in health and/or smart watches or other wrist-worn medical devices (or other health monitoring device patches or belts) that track activity (accelerometry), heart rate (PPG sensors) and/or respiration rate. Accordingly, all such modifications are intended to be included within the scope of the embodiments of the present disclosure as defined in the following claims. In the claims, means-plus-function clauses are intended to cover the structures described herein as performing the recited function and not only structural equivalents, but also equivalent structures.

In addition, any reference signs placed in parentheses in one or more claims shall not be construed as limiting the claims. The word "comprising" and "comprises," and the like, does not exclude the presence of elements or steps other than those listed in any claim or the specification as a whole. The singular reference of an element does not exclude the plural references of such elements and vice-versa. One or more of the embodiments may be implemented by means of hardware comprising several distinct elements, and/or by means of a suitably programmed computer. In a device claim enumerating several means, several of these means may be embodied by one and the same item of hardware. The mere fact that certain measures are recited in mutually different dependent claims does not indicate that a combination of these measures cannot be used to an advantage.

## Claims

1. A wearable device (10) for individual health assessment measurement of vital signs, the wearable device comprising:
a housing (16) having a bottom surface (22);
an optical sensor/emitter assembly (18) configured for emitting optical signals from the bottom surface (22) of the housing (16) through the skin of a subject and receiving reflected optical signals;
a controller (20) for controlling at least one physiological measurement modality of the wearable device in the measurement of the vital signs, wherein the controller (20 is coupled to the optical sensor/emitter assembly (18) for controlling the optical sensor/emitter assembly in transmitting and receiving optical signals; and
a light seal (12) configured to be removably coupled with the skin of the subject and to prevent ambient light interference with the optical sensor/emitter assembly when coupled, wherein the light seal comprises at least one integrated dry electrode (14) for at least one of (i) transmitting electrical signals to, (ii) receiving electrical signals from, and (iii) both transmitting and receiving electrical signals to/from the skin of the subject, the at least one integrated dry electrode being electrically coupled to the controller for obtaining electrical measurements according to the at least one physiological measurement modality during operation of the wearable device.

2. The wearable device (10) according to claim 1, wherein the light seal (12) comprises a physical configuration positioned about and extending from an outer perimeter of the bottom surface of the housing, wherein the light seal further includes a sidewall height dimension for spacing the optical sensor/emitter assembly (18) with respect to the skin of the subject.

3. The wearable device (10) according to claim 1, wherein the at least one integrated dry electrode (14) comprises multiple integrated dry electrodes, each separated from one another via electrically insulative material or an air gap.

4. The wearable device (10) according to claim 1, wherein one or more integrated dry electrode (14a, 14b) comprises a semi-circular shape when viewed from the bottom surface of the housing.

5. The wearable device (10) according to claim 1, wherein the light seal (12) comprises a rubber ring integrated with at least one dry electrode (14) that comprises a flexible, conductive polymer or elastomer.

6. The wearable device (10) according to claim 1, wherein the at least one physiological measurement modalities include one selected from the group consisting of electrocardiography (ECG), photoplethysmography (PPG), bioelectrical impedance analysis (BIA), and any combination thereof, to obtain physiological parameters that include one selected from the group consisting of heart rate (HR), pulse rate (PR), respiration rate (RR), SpO₂, stress, and any combination thereof.

7. The wearable device (10) according to claim 1, wherein the optical sensor/emitter assembly (18) comprises a photoplethysmography PPG assembly for deriving a PPG-based signal that includes heart rate HR and oxygen saturation SpO₂.

8. The wearable device (10) according to claim 1, further comprising a wearable band (28) or patch coupled to the housing for securing the housing to the skin of the subject while the wearable device is being worn by the subject.

9. The wearable device (10) according to claim 8, wherein the wearable band comprises one selected from the group consisting of a head band, wrist band, an ankle band, a chest band, and a waist band.

10. The wearable device (10) according to claim 1, further comprising at least one additional dry electrode (30,32) coupled to a side surface of the housing (16), other than the bottom surface, wherein the at least one additional dry electrode is electrically coupled to the controller (20) for obtaining electrical measurements according to the at least one physiological measurement modality during operation of the wearable device.

11. The wearable device (10) according to claim 10, wherein the at least one additional dry electrode comprises two additional dry electrodes (30,32), wherein the two additional dry electrodes are disposed on a same side surface of the housing.
